Europäisches Patentamt

European Patent Office

Office européen des brevets

(11) Publication number: **0 295 070**
**A2**

## EUROPEAN PATENT APPLICATION

(21) Application number: **88305214.4**

(22) Date of filing: **08.06.88**

(51) Int. Cl.⁴: **A 61 K 7/32**
**A 61 K 7/36, A 61 K 7/38**

(30) Priority: **11.06.87 US 61260**

(43) Date of publication of application:
**14.12.88 Bulletin 88/50**

(84) Designated Contracting States:
**AT BE CH DE FR GB IT LI LU NL**

(71) Applicant: **THE PROCTER & GAMBLE COMPANY**
**One Procter & Gamble Plaza**
**Cincinnati Ohio 45202 (US)**

(72) Inventor: **Tanner, Paul Robert**
**459 Dewdrop Circle, Apt. C.**
**Cincinnati Ohio 54240 (US)**

Luebbe, John Paul
R.R. 2, Meadowridge Drive
Lawrenceburg, IN 47025 (US)

Ward, Richard Martin
12139 Midpines Drive, Apt. 88
Cincinnati Ohio 45241 (US)

Juneja, Prem Sagar
7719 Stonehenge Drive
Cincinnati Ohio 45241 (US)

Zerby, Kim William
6363 Pawnee Ridge Drive
Loveland Ohio 45140 (US)

(74) Representative: **Brooks, Maxim Courtney et al**
**Procter & Gamble (NTC) Limited Whitley Road**
**Longbenton**
**Newcastle-upon-Tyne NE12 9TS (GB)**

(54) Liquid antiperspirant actives and processes for preparing the same.

(57) Liquid polyhydric alcohol solubilized antiperspirant actives comprising: at least one antiperspirant active selected from aluminum chlorhydroxide salts, zirconyl hydroxychloride salts, and mixtures thereof; at least one polyhydric alcohol; and water in specific amounts and relative proportions. These liquid antiperspirant actives have high concentrations of active and low amounts of water. They are stable, have good antiperspirant activity, are easy to manufacture and formulate, are cosmetically acceptable, and leave very little visible residue on skin and clothes.

EP 0 295 070 A2

## Description

# LIQUID ANTIPERSPIRANT ACTIVES AND PROCESSES FOR PREPARING THE SAME

## BACKGROUND OF THE INVENTION

The present invention relates to liquid polyhydric alcohol solubilized antiperspirant actives. These liquid antiperspirant actives have good antiperspirant activity and cosmetic aesthetics; and they are easily processed and formulated into antiperspirant compositions. The present invention also relates to processes for preparing the liquid polyhydric alcohol solubilized antiperspirant actives of the present invention. This invention further relates to antiperspirant compositions containing the liquid polyhydric alcohol solubilized antiperspirant actives of the present invention; and to methods for treating or preventing perspiration and malodor associated with human underarm perspiration.

A variety of references disclose antiperspirant actives which are soluble in non-aqueous solvents, typically monohydric alcohols (e.g., ethanol). U.S. Patent 3,873,686, to Beekman, issued March 25, 1975 (incorporated herein by reference in its entirety) provides a useful summary of work in this area. Additional references of particular interest in this area are the following which relate to antiperspirant actives soluble in non-aqueous solvents and/or to processes for making such actives: U.S. patent 3,359,169, to Slater et al., issued December 19, 1967; U.S. Patent 3,420,932, to Jones et al., issued January 7, 1969; U.S. Patent 3,507, 896, to Jones et al., issued April 21, 1970; U.S. Patent 3,523,130, to Jones et al., issued August 4, 1970; U. S. Patent 3,555,146, to Jones et al., issued January 12, 1971; U.S. Patent 3,876,758, to Beekman, issued April 8, 1975; Great Britain Patent Specifications 1,159,685 and 1,159,686, both published July 30, 1969, by Armour Pharmaceutical Company; and European Patent Application Publication Number 7,191, published January 23, 1980, by Unilever Limited.

Of particular interest is U.S. Patent 2,890,987, to Hilfer, issued June 16, 1959, which describes preparing relatively dilute solutions of astringent aluminum compounds in propylene glycol having relatively high water content. That these solutions have such characteristics (i.e., dilute actives and higher water content) is necessary in light of having been prepared by mixing a hot aqueous solution of the astringent aluminum compound (maximum solubility in water typically less than about 50%) with hot propylene glycol. Also of particular interest is U.S. Patent 4,137,306, to Rubino et al, issued January 30, 1979, which discusses the Hilfer U.S. Patent 2,890,987 and describes anhydrous propylene glycol solutions of alcohol soluble astringent basic aluminum compounds. Unlike the actives utilized in the present invention which are insoluble in anhydrous polyhydric alcohols, the aluminum compounds utilized in Rubino U.S. Patent 4,137,306 are those which are soluble in anhydrous alcohols.

Notwithstanding the large amount of work already performed in developing antiperspirant active compositions, there continues to be a need to develop new antiperspirant actives. An object of the present invention is to provide concentrated antiperspirant actives in liquid form having low water content. Another object of the present invention to provide liquid polyhydric alcohol solubilized antiperspirant actives having good antiperspirant activity. It is a further object to provide liquid antiperspirant actives which are stable and easy to manufacture and formulate. A still further object is to provide liquid antiperspirant actives which are cosmetically acceptable, and which leave very little visible residue on skin and clothes. Another object of the present invention is to provide processes for preparing the liquid antiperspirant actives of the present invention. A still further object of the present invention is to provide antiperspirant compositions utilizing these liquid antiperspirant actives, and to provide methods for treating or preventing human perspiration and underarm malodor by utilizing these liquid antiperspirant actives.

These and other objects will become readily apparent from the detailed description which follows.

All percentages and ratios used herein are by weight unless otherwise indicated.

## SUMMARY OF THE INVENTION

The present invention relates to liquid polyhydric alcohol solubilized antiperspirant actives. These liquid antiperspirant actives comprise:

(a) from about 30% to about 60% of at least one antiperspirant active selected from aluminum chlorhydroxide salts, zirconyl hydroxychloride salts, and mixtures thereof;

(b) from about 35% to about 55% of at least one polyhydric alcohol; and

(c) from about 10% to about 25% water; and wherein further the ratio of polyhydric alcohol to water is greater than about 3:2 and the ratio of antiperspirant active to water is greater than about 1.2:1.

The present invention further relates to processes for preparing liquid polyhydric alcohol solubilized antiperspirant actives. These processes comprise the steps of:

(a) combining at least one antiperspirant active selected from aluminum chlorhydroxide salts, zirconyl hydroxychloride salts, and mixtures thereof, with a polyhydric alcohol having a water content of less than about 40%, and wherein following this combination the ratio of antiperspirant active to polyhydric alcohol is greater than about 1:2 and the ratio of polyhydric alcohol to water is greater than about 3:2 and the ratio of antiperspirant active to water is greater than about 1.2:1;

(b) optionally adding water to this combination in an amount limited such that the ratio of polyhydric alcohol to water remains greater than about 3:2 and such that the ratio of antiperspirant active to water remains greater than about 1.2:1; and

(c) mixing the combination to form a liquid polyhydric alcohol solubilized antiperspirant active.

The present invention also relates to antiperspirant compositions comprising:

(a) from about 1% to about 75% of a liquid polyhydric alcohol solubilized antiperspirant active of the present invention; and

(b) an antiperspirant carrier.

The present invention finally relates to methods for treating or preventing perspiration and malodor associated with underarm perspiration. These methods comprise applying to the skin of a human a safe and effective amount of a liquid polyhydric alcohol solubilized antiperspirant active of the present invention.

## DETAILED DESCRIPTION OF THE INVENTION

### Liquid Polyhydric Alcohol Solubilized Antiperspirant Actives

The liquid antiperspirant actives of the present invention comprise specific amounts of the following: (a) polyhydric alcohol (preferably glycerine and/or propylene glycol); (b) antiperspirant active selected from aluminum chlorhydroxide, zirconyl hydroxychloride, and mixtures thereof; and (c) water.

The specific components of the liquid antiperspirant actives, and their levels, are selected in order to produce a liquid having the properties desired for the antiperspirant composition which the liquid antiperspirant active is to be formulated into. These components, and the specific weight percentages and relative ratios for these components, are described in detail immediately hereinafter.

### (a) Polyhydric Alcohols:

The polyhydric alcohols to be utilized in the present invention are one or more polyhydric alcohols selected such that the liquid antiperspirant actives of the present invention are liquids at room temperature. Typical liquid polyhydric alcohols for use in the liquid antiperspirant actives of the present invention include: 1,2-propylene glycol; 1,3-propylene glycol; 1,3-butylene glycol (1,3-butane-diol); glycerine (1,2,3-trihydroxy propane); 2-methyl-2,4-pentane-diol; and 2-ethyl-1,3-hexane-diol. Preferred for use herein is glycerine, propylene glycol and mixtures thereof. Most preferred is 1,2-propylene glycol.

The polyhydric alcohols of the present invention comprise, in total, from about 35% to about 55% of the liquid antiperspirant actives of the present invention, preferably from about 35% to about 50%, and most preferably from about 35% to about 45%.

### (b) Antiperspirant Actives:

Antiperspirant actives useful in the liquid polyhydric alcohol solubilized antiperspirant actives of the present invention are selected from aluminum chlorhydroxide salts, zirconyl hydroxychloride salts, and mixtures thereof; and especially those with enhanced efficacy due to improved molecular distributions.

Aluminum chlorhydroxide salts useful herein include those of the general formula:

$Al_2(OH)_aCl_b.xH_2O$

wherein a is from about 2 to about 5; $a + b = 6$; x is from about 1 to about 6; and wherein a, b, and x may have non-integer values. Particularly preferred are aluminum chlorhydroxides referred to as "5/6 basic chlorhydroxide", wherein $a = 5$; and "2/3 basic chlorhydroxide," wherein $a = 4$. Processes for preparing aluminum salts are disclosed in the following documents, all incorporated by reference herein in their entirety: U.S. Patent 3,887,692, to Gilman, issued June 3, 1975; and U.S. Patent 4,359,456, to Gosling et al., issued November 6, 1982.

Zirconyl hydroxychloride salts useful herein include those of the general formula:

$ZrO(OH)_{2-a}Cl_a.xH_2O$

wherein a is from about 1 to about 2, preferably from about 1.5 to about 1.87; x is from about 1 to about 7; and wherein a and x may have non-integer values. These zirconyl hydroxychloride salts are disclosed in Belgium Patent Specification 825,146, to Schmitz, issued August 4, 1975, the disclosures of which are incorporated herein by reference in their entirety.

Also useful herein are complexes containing zirconium, aluminum and glycine, commonly known as "ZAG complexes". Such ZAG complexes contain aluminum chlorhydroxide and zirconyl hydroxychloride to the formulae detailed above. These compounds in ZAG complexes are disclosed in the following patent documents, all incorporated by reference herein in their entirety: U.S. Patent 3,792,068, to Luedders et al., issued February 12, 1974; and U.S. Patent 4,120,948, to Shelton, issued October 17, 1978.

The most preferred antiperspirant actives useful in the liquid polyhydric alcohol solubilized antiperspirant actives of the present invention are antiperspirant actives with enhanced efficacy due to improved molecular distribution. Aluminum chlorhydroxide salts, zirconyl hydroxychloride salts, and mixtures thereof having improved molecular distributions are known, having been disclosed, for example, in the following documents, all incorporated by reference herein in their entirety: U.S. Patent 4,359,456, to Gosling et al, issued November 16, 1982; European Patent Application Publication No. 6,739, to Unilever Limited, published January 9, 1980; European Patent Application Publication No. 183,171, to Armour Pharmaceutical Company, published June 4, 1986; British Patent Specification 2,048,229, to The Gillette Company, published December 10, 1980; European Patent Application Publication No. 191,628, to Unilever PLC, published August 20, 1986; and British Patent Specification 2,144,992 to The Gillette Company, published March 20, 1985.

The improved molecular distribution is determined by the known analysis method called gel permeation chromatography. This analysis method is described, for example, in several of the above-incorporated patent specification as well as in European Patent Application Publication No. 7,191, to Unilever Limited, published January 23, 1980, the disclosures of which are incorporated herein by reference in their entirety. It is preferred for purposes of the present invention that the liquid polyhydric alcohol solubilized antiperspirant actives have enhanced efficacy due to improved molecular distribution having the ratio of peak 3 to peak 2 greater than about 0.1:1 as determined by gel permeation chromotography. This ratio, as is recognized by one skilled in the art, relates to the relative area under these two peaks as measured by the gel permeation chromatography analysis method.

The antiperspirant actives useful in the liquid antiperspirant active compositions of the present invention are not soluble in anhydrous polyhydric alcohols. However, when formulated according to the present invention by including a surprisingly small amount of water with the polyhydric alcohol these antiperspirant actives are soluble at high concentrations in the polyhydric alcohol to form a liquid composition. The terms "soluble" and "solubilized", as used herein, mean that the antiperspirant active is dissolved in and/or colloidally dispersed (sub-micron particle size) in the polyhydric alcohol and water of the liquid antiperspirant active composition to give a transparent or semi-transparent liquid. Typically the transparency of the liquid is such that more than 50%, preferably more than 75%, of 500 nm light is transmitted through the liquid as measured by a standard UV-visible absorption instrument (relative to aqueous polyhydric alcohol without antiperspirant active).

The antiperspirant actives utilized herein comprise in total from about 30% to about 60% of the liquid antiperspirant active composition of the present invention, preferably from about 30% to about 55%, and most preferably from about 35% to about 50%. These weight percentages exclude any amount of water, either unbound or bound by the antiperspirant active, which is detectable by Karl-Fisher moisture analysis. All amounts of water present in the liquid antiperspirant actives which are detectable by this analysis method are therefore to be considered to be part of the water component of the liquid antiperspirant active.

(c) Water

The final essential component of the liquid antiperspirant active of the present invention is water. Water is present at a level of from about 10% to about 15%, preferably from about 10% to about 20%, and most preferably from about 13% to about 20%. This amount of water includes all water present in the solution which is detectable by Karl-Fisher moisture analysis, whether or not bound to or complexed with the antiperspirant active.

Furthermore, the ratio of polyhydric alcohol to water is greater than about 3:2, and preferably greater than about 2:1. Typically this ratio is within the range of from about 3:2 to about 4:1, and preferably within the range of from about 2:1 to about 3:1. In addition, the ratio of antiperspirant active to water is greater than about 1.2:1, and preferably greater than about 1.5:1. Typically this ratio is within the range of from about 1.2:1 to about 6:1, and preferably within the range of from about 1.5:1 to about 3:1.

Antiperspirant Compositions

The present invention also relates to antiperspirant compositions, preferably in stick form. These antiperspirant compositions comprise at least one liquid antiperspirant active as described hereinbefore, and at least one antiperspirant carrier, preferably carriers for antiperspirant in stick form.

Antiperspirant carriers are well-known in the art, and the selection of antiperspirant carriers for use in the antiperspirant compositions of the present invention can be readily made by one skilled in the art. Antiperspirant carriers include hardeners, strengtheners, chelating agents, emollients, colorants, perfumes, emulsifiers, and fillers. The preferred antiperspirant compositions in stick form also essentially comprise solidifying agents as described more fully hereinafter. Antiperspirant carrier components are described more fully in the following publications, the disclosures of which are incorporated by reference herein in their entirety. Plechner, "Antiperspirants and Deodorants", Cosmetics, Science and Technology, 2, pages 373-416 (Balsam and Sagarin, editors; 1972); Fox, "Gel and Sticks Review and Update", Cosmetics and Toiletries, 99, pages 19-52 (1984); Geria, "Formulation of Stick Antiperspirants and Deodorants", Cosmetics and Toiletries, 99, pages 55-99 (1984); and "Gels and Sticks Formulary", Cosmetics and Toiletries, 99, pages 77-87 (1984). These antiperspirant carriers may also include deodorant actives and/or other antiperspirant actives. The most preferred antiperspirant carriers for use herein are described in copending U.S. Patent Application Serial No. (P&G Case 3673) by Tanner et al., "Low Residue Wax Emulsion Antiperspirant Sticks", filed June 11, 1987 (incorporated herein by reference in its entirety).

Solidifying agents preferred for use in the antiperspirant compositions in stick form of the present invention are waxy materials typically incorporated at a level of from about 1% to about 35%, preferably from about 10% to about 30%. Among such waxy materials useful herein are the high melting point waxes having a melting point of from about 65°C to 102°C, low melting point waxes having a melting point of from about 37°C to 75°C, and preferably mixtures thereof. High melting point waxes include beeswax, spermaceti, carnauba, baysberry, candelilla, montan, ozokerite, ceresin, paraffin, hydrogenated castor oil (castor wax), synthetic waxes such as Fisher-Tropsch waxes, microcrystalline waxes, and mixtures thereof. Castor wax is a preferred high-melting point wax useful herein. Low melting point waxes include fatty acids, fatty alcohols, fatty acid esters, and fatty acid amides, having fatty chains of from about 8 to about 30 carbon atoms, preferably from about 12 to about 18 carbon atoms, and mixtures thereof. Preferred low melting point waxes include cetyl alcohol, palmitic acid, cetyl stearate, cetyl palmitate, and mixtures thereof. Solidifying agents among those useful in wax type antiperspirant sticks of this invention are disclosed in the following patent specifications, all of which are incorporated by reference herein in their entirety: U.S. Patent 4,049,792, to Elsnau, issued September 20, 1977; U.S. Patent 4,151,272, to Geary et al., issued April 24, 1975; U.S. Patent 4,229,432, to Geria, issued October 21, 1980; U.S. Patent 4,280,994, to Turney, issued July 28, 1981; U.S. Patent 4,126,679, to Davy et al., issued November 21, 1978; and European Patent Application Publication Number 117,070, to May, published August 29, 1984.

The liquid antiperspirant actives of the present invention typically comprised in total from about 1% to about 75%, preferably from about 10% to about 65%, and most preferably from about 30% to about 55%, of the compositions of the present invention. The antiperspirant carriers typically comprised in total from about 25% to about 99%, preferably from about 35% to about 90%, and most preferably from about 45% to about 70%, of the compositions of the present invention.

## Methods for Preventing Perspiration and Malodor

The present invention also provides methods for treating or preventing perspiration and malodor associated with human underarm perspiration. These methods comprise applying to the skin of a human a safe and effective amount of a liquid antiperspirant active of the present invention. The term "a safe and effective amount", as used herein, is an amount which is effective in eliminating or substantially reducing perspiration and malodor associated with human underarm perspiration while being safe for human use at a reasonable risk/benefit ratio.

## Process for Preparing Liquid Antiperspirant Actives

The present invention further relates to processes for preparing liquid antiperspirant actives of the present invention. These processes comprise the steps of:

(a) combining at least one antiperspirant active selected from aluminum chlorhydroxide salts, zirconyl hydroxychloride salts, and mixtures thereof with a polyhydric alcohol having a water content of less than about 40% (preferably less than about 30%; more preferably less than about 25%), wherein following this combination the ratio of antiperspirant active to polyhydric alcohol is greater than about 1:2 and the ratio of polyhydric alcohol to water is greater than about 3:2 (preferably greater than about 2:1) and the ratio of antiperspirant active to water is greater than about 1.2:1 (preferably greater than about 1.5:1; more preferably greater than about 2:1);

(b) optionally adding water to this combination in an amount limited such that the ratio of polyhydric alcohol to water remains greater than about 3:2 and such that the ratio of antiperspirant active to water remains greater than about 1.2:1; and

(c) mixing the combination to form a liquid polyhydric alcohol solubilized antiperspirant active.

Typically, the mixing of the components to form the liquid polyhydric alcohol solubilized antiperspirant active is performed at temperatures within the range of from about 20°C to about 100°C.

The following examples further describe and demonstrate embodiments within the scope of the present invention. The examples are given solely for the purpose of illustration and are not to be construed as limitations of the present invention as many variations thereof are possible without departing from the spirit or scope.

## EXAMPLE I

### Propylene Glycol Solubilized ZAG

Propylene glycol (177.6 g; U.S.P. supplied by Union Carbide) and water (44.8 g) are mixed in a beaker. To this solution is added aluminum zirconium tetrachlorohydrex glycine powdered active (177.6 g; improved molecular distribution supplied by Wickhen; approximately 10% water) while milling the mixture with a Tekmar Tissuemizer (slight warming results from milling). The resulting clear liquid retains an improved molecular distribution (ratio of peak 3 to peak 2 of 1.1:1), and contains 40% ZAG, 44% propylene glycol, and 16% water.

## EXAMPLE II

### Propylene Glycol Solubilized ZAG

Propylene glycol (177.6 g; U.S.P. supplied by Union Carbide) and water (44.8 g) are mixed in a beaker and then heated to 80°C. To this solution is slowly added aluminum zirconium trichlorohydrex glycine powdered active (177.6 g; improved molecular distribution supplied by Westwood; approximately 10% water) while milling the mixture with a Tekmar Tissuemizer. The resulting clear liquid retains an improved molecular distribution (ratio of peak 3 to peak 2 of 0.5:1) and contains 40% ZAG, 44% propylene glycol, and 16% water. This composition is also prepared without milling by very slowly adding the ZAG powder in small quantities while stirring, allowing each addition to dissolve before making the next addition.

A clear liquid propylene glycol solubilized ZAG (45% ZAG, 40% propylene glycol, and 15% water) is prepared by the same procedure as above using 160 g propylene glycol, 40 g water, and 200 g ZAG active. This clear liquid propylene glycol solubilized ZAG is also prepared as follows: First mix together the 160 g of propylene glycol and 200 g of ZAG, and then heat to 80°C and mill to disperse the powder. While milling, 40 g of water is slowly added to convert the suspension into the clear liquid propylene glycol solubilized ZAG.

## EXAMPLE III

### Propylene Glycol Solubilized ACH

Propylene glycol (177.6 g; U.S.P. supplied by Union CArbide) and water (44.8 g) are mixed in a beaker and then heated to 80°C. To this solution is slowly added aluminum chlorhydroxide powdered active (177.6 g; improved molecular distribution supplied by Westwood; approximately 20% water ) while milling the mixture with a Tekmar Tissuemizer. The resulting clear liquid retains an improved molecular distribution (ratio of peak 3 to peak 2 of 0.6:1), and contains 36% ACH, 44% propylene glycol, and 20% water.

## EXAMPLE IV

### Propylene Glycol Solubilized ZAG

Propylene glycol (8000 g; U.S.P. supplied by Union Carbide) and water (2000 g) are mixed together and then heated to 50°C. While milling with a Tekmar lab mill, aluminum zirconium trichlorohydrex glycine powdered active (10,000 g; improved molecular distribution supplied by Westwood; approximately 10% water) is slowly added and the milling continued until all the powder is dissolved. The resulting clear liquid retains an improved molecular distribution (ratio of peak 3 to peak 2 of 0.3:1), and contains 45% ZAG, 40% propylene glycol, and 15% water.

## EXAMPLE V

### Glycerine Solubilized ZAG

Glycerine (150 g) and water (30 g) are mixed in a beaker and then heated to 50°C. To this solution is added aluminum zirconium trichlorohydrex glycine powdered active (120 g; improved molecular distribution supplied by Westwood; approximately 10% water) while milling the mixture with a Tekmar Tissuemizer. The resulting clear liquid retains an improved molecular distribution and contains 45% ZAG, 40% glycerine, and 15% water.

## EXAMPLE VI

An antiperspirant composition of the present invention in stick form is prepared comprising the following ingredients:

| Components | Weight % |
|---|---|
| Cyclomethicone D-5[1] | 26.0 |
| Isohexadecane[2] | 5.0 |
| Dow Corning Q2-3225C[3] | 1.0 |
| Cetyl Stearate[4] | 22.0 |
| Castor Wax MP70[5] | 1.0 |
| Propylene Glycol Solubilized ZAG[6] | 45.0 |

[1] Supplied by GE Silicones

[2] Permethyl 101A supplied by Presperse and made by Permethyl Corp.

[3] A mixture of cyclomethicone and dimethicone copolyol (ratio of approximately 9:1) supplied by Dow Corning

[4] Schercemol CS supplied by Scher Chemicals

[5] Hydrogenated castor oil supplied by CAS Chemicals

[6] Prepared as described in Example IV.

This stick composition is prepared as follows. All the ingredients except the propylene glycol solubilized ZAG are combined and heated to 180°F (82°C). The solubilized active is then prepared by milling the ZAG into a 122°F (50°C) mixture of the water and propylene glycol until all the ZAG powder is dissolved. The oil and wax mixture is then cooled to 117°F (47°C) and the warm solubilized active is slowly added with milling. This composition is cooled with agitation to 113°F (45°C) and poured into canisters. A uniform antiperspirant stick forms as the composition cools to room temperature.

This antiperspirant composition is applied to the underarm skin of a human to effectively prevent perspiration and underarm odor resulting from perspiration. The composition is relatively non-sticky and glides on the skin smoothly. It also produces relatively little visible white residue on the skin and clothes, both initially upon application and later.

**Claims**

1. Liquid polyhydric alcohol solubilized antiperspirant actives comprising:
(a) from about 30% to about 60% of at least one antiperspirant active selected from the group consisting of aluminum chlorhydroxide salts, zirconyl hydroxychloride salts, and mixtures thereof;
(b) from about 35% to about 55% of at least one polyhydric alcohol; and
(c) from about 10% to about 25% water;
and wherein further the ratio of polyhydric alcohol to water is greater than about 3:2 and the ratio of antiperspirant active to water is greater than about 1.2:1.

2. Liquid polyhydric alcohol solubilized antiperspirant actives, according to Claim 1, wherein the polyhydric alcohol is selected from the group consisting of propylene glycol, glycerine, and mixtures thereof.

3. Liquid polyhydric alcohol solubilized antiperspirant actives, according to Claim 1, wherein the antiperspirant active has enhanced efficacy due to improved molecular distribution having the ratio of peak 3 to peak 2 greater than about 0.1:1 as determined by gel permeation chromatography.

4. Liquid polyhydric alcohol solubilized antiperspirant actives, according to Claim 3, wherein the polyhydric alcohol is selected from the group consisting of propylene glycol, glycerine, and mixtures thereof.

5. Liquid polyhydric alcohol solubilized antiperspirant actives, according to Claim 4, wherein the antiperspirant active is selected from the group consisting of aluminum chlorhydroxide salts,

7

zirconium-aluminum-glycine complexes, and mixtures thereof.

6. Liquid polyhydric alcohol solubilized antiperspirant actives comprising:

(a) from about 30% to about 55% of at least one antiperspirant active selected from the group consisting of aluminum chlorhydroxide salts, zirconyl hydroxychloride salts, and mixtures thereof;

(b) from about 35% to about 50% of at least one polyhydric alcohol selected from the group consisting of propylene glycol, glycerine, and mixtures thereof; and

(c) from about 10% to about 20% water;

and wherein further the ratio of polyhydric alcohol to water is greater than about 3:2 and the ratio of antiperspirant active to water is greater than about 1.5:1.

7. Liquid polyhydric alcohol solubilized antiperspirant actives, according to Claim 6, wherein the antiperspirant active has enhanced efficacy due to improved molecular distribution having the ratio of peak 3 to peak 2 greater than about 0.1:1 as determined by gel permeation chromatography.

8. Liquid polyhydric alcohol solubilized antiperspirant actives, according to Claim 7, wherein the antiperspirant active is selected from the group consisting of aluminum chlorhydroxide salts, zirconium-aluminum-glycine complexes, and mixtures thereof.

9. Liquid polyhydric alcohol solubilized antiperspirant actives comprising:

(a) from about 35% to about 50% of at least one antiperspirant active selected from the group consisting of aluminum chlorhydroxide salts, zirconyl hydroxychloride salts, and mixtures thereof;

(b) from about 35% to about 45% of at least one polyhydric alcohol selected from the group consisting of propylene glycol, glycerine, and mixtures thereof; and

(c) from about 13% to about 20% water;

and wherein further the ratio of polyhydric alcohol to water is within the range of from about 3:2 to about 4:1 and the ratio of antiperspirant active to water is within the range of from about 1.5:1 to about 3:1.

10. Liquid polyhydric alcohol solubilized antiperspirant actives, according to Claim 9, wherein the antiperspirant active has enhanced efficacy due to improved molecular distribution having the ratio of peak 3 to peak 2 greater than about 0.1:1 as determined by gel permeation chromatography.

11. Liquid polyhydric alcohol solubilized antiperspirant actives, according to Claim 10, wherein the polyhydric alcohol comprises 1,2-propylene glycol.

12. Antiperspirant compositions comprising:

    (a) from about 1% to about 75% of a liquid polyhydric alcohol solubilized antiperspirant active according to Claim 1; and

    (b) an antiperspirant carrier.

13. Antiperspirant compositions comprising:

    (a) from about 1% to about 75% of a liquid polyhydric alcohol solubilized antiperspirant active according to Claim 6; and

    (b) an antiperspirant carrier.

14. Antiperspirant compositions comprising:

    (a) from about 1% to about 75% of a liquid polyhydric alcohol solubilized antiperspirant active according to Claim 9; and

    (b) an antiperspirant carrier.

15. Antiperspirant compositions comprising:

    (a) from about 1% to about 75% of a liquid polyhydric alcohol solubilized antiperspirant active according to Claim 11; and

    (b) an antiperspirant carrier.

16. Methods for treating or preventing perspiration and malodor associated with human underarm perspiration, said methods comprising applying to the skin of a human a safe and effective amount of a liquid polyhydric alcohol solubilized antiperspirant active according to Claim 1.

17. Methods for treating or preventing perspiration and malodor associated with human underarm perspiration, said methods comprising applying to the skin of a human a safe and effective amount of a liquid polyhydric alcohol solubilized antiperspirant active according to Claim 9.

18. Methods for treating or preventing perspiration and malodor associated with human underarm perspiration, said methods comprising applying to the skin of a human a safe and effective amount of a liquid polyhydric alcohol solubilized antiperspirant active according to Claim 11.

19. Processess for preparing liquid polyhydric alcohol solubilized antiperspirant actives according to Claim 1, said processess comprising the steps of:

    (a) combining at least one antiperspirant active selected from the group consisting of aluminum chlorhydroxide salts, zirconyl hydroxychloride salts, and mixtures thereof, with a polyhydric alcohol having a water content of less than about 40%, and wherein following this combination the ratio of antiperspirant active to polyhydric alcohol is greater than about 1:2 and the ratio of polyhydric alcohol to water is greater than about 3:2 and the ratio of antiperspirant active to water is greater than about 1.2:1;

    (b) optionally adding water to this combination in an amount limited such that the ratio of polyhydric alcohol to water remains greater than about 3:2 and such that the ratio of antiperspirant active to water remains greater than about 1.2:1; and

    (c) mixing the combination to form a liquid polyhydric alcohol solubilized antiperspirant active.

20. Processess for preparing liquid polyhydric alcohol solubilized antiperspirant actives according to Claim 10, said processess comprising the steps of:

(a) combining at least one antiperspirant active, having enhanced efficacy due to improved molecular distribution having the ratio of peak 3 to peak 2 greater than 0.1:1 as determined by gel permeation chromatography, selected from the group consisting of aluminum chlorhydroxide salts, zirconyl hydroxychloride salts, and mixtures thereof, with a polyhydric alcohol having a water content of less than about 30%, and wherein following this combination the ratio of antiperspirant active to polyhydric alcohol is greater than about 1:2 and the ratio of polyhydric alcohol to water is greater than about 2:1 and the ratio of antiperspirant active to water is greater than about 1.5:1;

(b) optionally adding water to this combination in an amount limited such that the ratio of polyhydric alcohol to water remains greater than about 2:1 and such that the ratio of antiperspirant active to water remains greater than about 1.5:1; and

(c) mixing the combination to form a liquid polyhydric alcohol solubilized antiperspirant active.